# EUROPÄISCHE PATENTSCHRIFT

(11) **EP 3 436 102 B1**
(45) Veröffentlichungstag und Bekanntmachung des Hinweises auf die Patenterteilung: **24.07.2024**
(21) Anmeldenummer: 17714194.2
(22) Anmeldetag: 28.03.2017
(51) Int. Cl.: A61M 1/00, A61N 1/44, A61L 2/14, A61B 18/04

(54) **SYSTEM UND VERFAHREN ZUR BEHANDLUNG VON OBERFLÄCHEN VON KÖRPERN, INSBESONDERE ZUR WUNDBEHANDLUNG**
SYSTEM AND METHOD FOR TREATING SURFACES OF BODIES, IN PARTICULAR FOR WOUND TREATMENT
SYSTÈME ET MÉTHODE POUR TRAITER DES SURFACES DE CORPS, EN PARTICULIER POUR TRAITER DES PLAIES

(30) Priorität: 30.03.2016 DE 102016105759
(43) Veröffentlichungstag der Anmeldung: 06.02.2019
(73) Patentinhaber: Leibniz-Institut für Plasmaforschung und Technologie e.V., 17489 Greifswald (DE)
(72) Erfinder: WELTMANN, Klaus-Dieter, 18609 Binz (DE); VON WOEDTKE, Thomas, 18519 Sundhagen (DE); STIEBER, Manfred, 17489 Greifswald (DE); HORN, Stefan, 17509 Loissin (DE); TURSKI, Philipp, 17489 Greifswald (DE); BRANDENBURG, Ronny, 17495 Groß Kiesow OT Kessin (DE)
(74) Vertreter: Schulz Junghans Patentanwälte PartGmbB
(86) Internationale Anmeldenummer: PCT/EP2017/057310
(87) Internationale Veröffentlichungsnummer: WO 2017/167748

(56) Entgegenhaltungen:
- EP-A1- 2 170 022
- WO-A1-2008/138504
- DE-A1- 102011 105 713
- DE-A1- 102013 113 905

## Beschreibung

Die Erfindung betrifft ein System zur Behandlung von Oberflächen von Körpern, insbesondere zur Wundbehandlung mittels kombinierter Plasma- und Unterdruckbehandlung, sowie ein entsprechendes Verfahren zur Behandlung von Oberflächen von Gewebe. Dabei handelt es sich bei dem System insbesondere um einen Vakuumverband zur kombinierten Plasma- und Unterdruckbehandlung.

Die Behandlung von komplizierten bzw. chronischen Wunden mittels der Unterdruckwundtherapie (auch "Vakuumtherapie", "Negative pressure wound therapy, NPWT" oder "Vacuum-Assisted-Closure, V.A.C." genannt) wurde erstmals 1991 in den Patentschriften der Wake Forest University Winston-Salem durch Louis C. Argenta und Michael J. Morykwas beschrieben (z.B. WO 9309727 A1 und WO 9420041 A1). Es folgten zahlreiche weitere Patentschriften und Publikationen dieser und anderer Autoren, in denen Weiterentwicklungen dieser Methode und deren Anwendungen beschrieben werden. Bei dieser Applikation wird die Wunde einem Unterdruck ausgesetzt, wodurch krankhaftes Wundsekret abgesaugt und die Wundheilung stimuliert oder unterstützt wird. Es werden dabei durch entsprechend umgesetzte Geräte vor allem folgende Wirkungen erzielt, die zu einer verbesserten Heilung chronischer Problemwunden beitragen:
- Reduktion der Flüssigkeitsansammlung im Gewebezwischenraum (Wund-Ödem),
- Verbesserung der Sauerstoffsättigung im Gewebe,
- Verbesserung der lokalen Durchblutungssituation,
- Verminderung bakterieller Keimvermehrung (Kolonisation),
- Reduktion der Menge an zellulären Zerfallsprodukten (Zelldetritus),
- Stimulation des Zellwachstums durch mechanischen Stress,
- Förderung der Granulationsgewebsbildung.

Die Unterdruckwundtherapie ist insbesondere in den folgenden Veröffentlichungen beschrieben:
- Argenta LC, Morykwas MJ (1997) Vacuum-assisted closure: a new method for wound control and treatment: clinical experience. Ann Plast Surg 38: 563-76
- Morykwas MJ, Argenta LC, Shelton-Brown EI (1997) Vacuum-assisted closure: a new method for wound control and treatment: animal studies and basic foundation. Ann Plast Surg 38:553-62
- Horch, R. and M. Leffler, Grundlagen, Indikationen, grundlegende therapeutische Konzepte und Kontraindikationen bei der Wundbehandlung mit der Vakuumtherapie, in Manual der Wundheilung: Chirurgisch-dermatologischer Leitfaden der modernen Wundbehandlung, T. Wild, Auböck, Josef (Hrsg.) Editor. 2007. p. 123-128.
- Horch, R.E., et al., Lokale Unterdrucktherapie im Wundmanagement, in EWMA-Positionsdokument 2007, European Wound Management Association (EWMA). London: MEP Ltd,. p. 1-17.
- Probst, W. and A. Vasel-Biergans, 10. Besondere Verfahren der Wundbehandlung; 10.1 Lokale Unterdrucktherapie, in Wundversorgung für die Pflege - Ein Praxisbuch; 2011, Wissenschaftliche Verlagsgesellschaft p. 319-326.
- Protz, K. Tiefe Wunden und Wundhöhlen, Der Hausarzt 08/2014, S.52-54.

Die in verschiedenen Studien und Fallberichten beschriebenen Erfolge in der Behandlung schwer heilender Wunden sowie der Nachweis der Wirtschaftlichkeit der Unterdrucktherapie gegenüber konventionellen Methoden der Wundbehandlung bei schwierigen Wundproblemen, vor allem durch die Verkürzung der für die Wundheilung und Pflege erforderlichen Zeiten, haben dazu geführt, dass sich diese Methode auf der Grundlage kommerziell verfügbarer Systemen im klinischen Alltag etabliert hat.

Ein derartiges Unterdrucktherapie-System besteht in der Regel aus einem Vakuumverband und einem Steuergerät mit einer Vakuumpumpe zur kontrollierten Erzeugung des Unterdruckes. Der Vakuumverband ist eine Kombination aus einer Schaumstoff-Wundauflage (aus Polyurethan oder Polyvinyl), einer transparenten Folie zur Abdichtung und Abdeckung des Vakuumverbandes sowie einer, an dieser Folie angeschlossenen, Saug-Drainage. Mit dem Vakuumsystem kann kontinuierlich oder gepulst ein definierter Unterdruck erzeugt werden. Komplettiert wird das System von einem Auffangbehälter zur Aufnahme der abgesaugten Wundflüssigkeit.

Die durch den Stand der Technik bekannten Lösungen haben insbesondere den Nachteil, dass zwar eine verminderte bakterielle Keimvermehrung, aber ohne eine zusätzliche Instillationsbehandlung (wie in den Patentdokumenten DE 19 722 075 C1 und DE 10 2013 226 708 A1 beschrieben) keine deutliche Keimreduktion im Wundbereich erzielt wird.

Ein weiterer Nachteil besteht darin, dass zur Beurteilung des Wundzustandes bzw. des Behandlungserfolges der Vakuumverband geöffnet werden muss und so einerseits der ungestörte Wundheilungsprozess beeinträchtigt wird und andererseits die Gefahr einer weiteren Infektion besteht. Hinzu kommt, dass bei einer Vielzahl von Wunden (z.B. Brandwunden) der Verbandswechsel den Patienten große Schmerzen bereitet.

Die EP 2 170 022 A1 offenbart ein System zur Behandlung von Oberflächen von Körpern, insbesondere einen Vakuumverband. Das System umfasst eine Anschlusseinrichtung zum strömungstechnischen Anschluss einer Unterdruckerzeugungseinrichtung zur Erzeugung eines Unterdrucks in einem an der Oberfläche eines Körpers positionierbaren Volumen. Weiterhin umfasst das System eine Einrichtung zur Plasmaerzeugung, mit der das Volumen, in welchem der Unterdruck erzeugbar ist, zumindest teilweise mit Plasma oder einem plasmaaktivierten Medium gefüllt werden kann.

Hieraus ergibt sich die Aufgabe, ein System und ein entsprechendes Verfahren zur Behandlung von Oberflächen von Körpern zur Verfügung zu stellen, das bezüglich der beschriebenen Nachteile des Standes der Technik verbessert ist.

Diese Aufgabe wird durch das System nach Anspruch 1 und das Verfahren nach Anspruch 14 gelöst. Vorteilhafte Ausgestaltungen des Systems sind Gegenstand der Unteransprüche 2 bis 13 und eine vorteilhafte Ausgestaltung des Verfahrens ist in dem Unteranspruch 15 beansprucht. Die Erfindung wird im Folgenden beschrieben.

Ein erster Aspekt der Erfindung betrifft ein System zur Behandlung von Oberflächen von Körpern, insbesondere zur Wundbehandlung, umfassend wenigstens eine Anschlusseinrichtung zum strömungstechnischen Anschluss einer Unterdruckerzeugungseinrichtung zur Erzeugung eines Unterdrucks in einem an der Oberfläche eines Körpers positionierbaren Volumen sowie wenigstens eine Einrichtung zur Plasmaerzeugung, mit der das Volumen, in welchem der Unterdruck erzeugbar ist, zumindest teilweise mit Plasma oder einem plasmaaktivierten Medium gefüllt werden kann.

Das System weist eine erste Einrichtung zur Plasmaerzeugung auf, die in dem Volumen, in welchem der Unterdruck erzeugbar ist, angeordnet ist, so dass das Plasma und/oder plasmaaktivierte Medium in dem Volumen erzeugbar ist. Die erste Einrichtung zur Plasmaerzeugung ist als ein Sensorsystem zur Erfassung wenigstens eines physikalischen Parameters an der Oberfläche des Körpers eingerichtet, wobei die Plasmaquelle der ersten Einrichtung zur Plasmaerzeugung zusätzlich als Sensorsystem für Wund-Monitoring verwendet werden kann.

Das Volumen kann dabei durch eine geeignete, von dem System umfasste Kammer oder durch ein poröses Material wie z.B. Schaumstoff, in dem der Unterdruck ausgebildet wird, definiert werden. In ergänzender Ausgestaltung umfasst das System zur Behandlung von Oberflächen von Körpern auch die Unterdruckerzeugungseinrichtung.

Bei dem erfindungsgemäßen System handelt es sich insbesondere um einen Vakuumverband zur Behandlung einer Wunde einer menschlichen oder tierischen Körperoberfläche.

Insbesondere wird erfindungsgemäß ein Gerät zur Unterdruckwundtherapie mit einer antimikrobiell und wundheilend wirkenden Atmosphärendruck-Plasmaquelle, sowie optional mit einer Vorrichtung zum sensorbasierten Wund-Monitoring, zu einem neuartigen Gerätesystem kombiniert.

Hierfür kann z.B. ein Gerät zur Unterdruckwundtherapie mit einer flächigen Plasmaquelle zur Erzeugung einer dielektrisch behinderten Entladung (DBE), die gemeinsam mit SchaumstoffWundauflagen des Gerätes zur Unterdruckwundtherapie in einer Sandwich-Anordnung im Wundbereich zu positionieren ist, kombiniert werden. Die dafür verwendete Plasmaquelle zur Erzeugung einer dielektrisch behinderten Entladung ist vorzugsweise aus flexiblen Materialien gefertigt, damit sie sich zusammen mit den Wundauflagen an die Oberfläche der Wunde anschmiegen kann.

Das erfindungsgemäße System ermöglicht eine wesentlich effizientere Behandlung von komplizierten bzw. chronischen Wunden gegenüber der Anwendung eines konventionellen Unterdruckwundtherapie-Gerätes. Dabei ist die Anwendung des erfindungsgemäßen Systems sowie des damit durchführbaren Verfahrens nicht auf medizinische Zwecke beschränkt, sondern es kann überall dort eingesetzt werden, wo eine effektive und effiziente Keimminderung gefordert ist.

Insbesondere die Einrichtung zur Unterdruckerzeugung des erfindungsgemäßen Systems kann in herkömmlicher Weise ausgestaltet sein, so dass diesbezüglich auf die erwähnten Veröffentlichungen verwiesen wird, deren auf den Unterdruck gerichteten Offenbarungsgehalte hiermit in die vorliegende Patentanmeldung einbezogen sind.

Neben der, durch die konventionelle Unterdrucktherapie erzielte verminderte bakterielle Keimvermehrung wird durch die zusätzlich verwendete Plasmaquelle einerseits eine deutliche Keimreduktion im Wundbereich und andererseits eine Förderung der Wundheilung erzielt. Insbesondere können durch die Kombination eines Gerätes zur Unterdrucktherapie mit einem Gerät zur Erzeugung einer Plasmaentladung MRSA-Infektionen wirksam vermieden werden.

Ein weiterer Vorteil besteht darin, dass durch die Nutzung des integrierten Sensorsystems zur Beurteilung des Wundzustandes bzw. des Behandlungserfolges der Vakuumverband nicht vorzeitig geöffnet werden muss und so einerseits der Wundheilungsprozess ungestört ablaufen kann und andererseits die Gefahr einer weiteren Infektion reduziert wird.

Die Anschlusseinrichtung des erfindungsgemäßen Systems zur Behandlung von Oberflächen von Körpern kann beispielsweise über eine Schlauchleitung, z.B. eines Drainageschlauches, mit einer Unterdruckerzeugungseinrichtung, wie z.B. einer Vakuumpumpe, strömungstechnisch verbunden sein.

Mit dem erfindungsgemäßen System zur Behandlung von Oberflächen von Körpern lässt sich somit das Plasma bzw. plasmaaktiviertes Medium direkt an der zu behandelnden Oberfläche erzeugen.

Der physikalische Parameter kann insbesondere auf der Oberfläche oder im Projektionsbereich der Oberfläche, aber auch in dem an der Oberfläche des Körpers positionierten Volumen erfasst werden. Mittels des auf diese Weise erfassten physikalischen Parameters können insbesondere Rückschlüsse auf den Zustand einer Wunde des Körpers, an dem das System zur Behandlung von Oberflächen von Körpern angeordnet ist, gezogen werden, z.B. im Rahmen eines Wund-Monitoring-Prozesses. Geeignete physikalische Parameter sind hierfür insbesondere Temperatur, Druck, Feuchtigkeit und/oder pH-Wert.

Das bedeutet, dass die erste Einrichtung zur Plasmaerzeugung eine Doppelfunktion erfüllt, nämlich die Erzeugung von Plasma bzw. plasmaaktiviertem Medium an der zu behandelnden Oberfläche sowie die Ermittlung wenigstens eines physikalischen Parameters an der Oberfläche des Körpers, wobei je Zeiteinheit lediglich eine dieser Funktionen erfüllt wird.

Grundlage der Messung von Parametern zur Beurteilung des Wundzustandes, wie Temperatur, Feuchte, Druck und pH-Wert, ist die Erfassung und Verarbeitung der Änderungen von elektrischen Gleich- oder Wechselstromgrößen (ohmscher Widerstand, Impedanz, Kapazität) an den elektrisch leitenden Komponenten Außenelektrode und/ oder Innenelektrode.

Insbesondere ist das erfindungsgemäße System zur Behandlung von Oberflächen von Körpern dazu eingerichtet, von einem aktiven Plasmaerzeugungs-Modus in einen Sensor-Modus umzuschalten, z.B. mittels einer mikroprozessor-basierten Prozesssteuerung.

Weiterhin kann das System zur Behandlung von Oberflächen von Körpern eine zweite Einrichtung zur Plasmaerzeugung aufweisen, die in Bezug zu dem Volumen, in welchem der Unterdruck erzeugbar ist, extern angeordnet ist und mittels einer strömungstechnischen Verbindung mit dem Volumen verbunden ist, so dass das Plasma oder das plasmaaktivierte Medium bzw. plasmaaktiviertes Medium außerhalb des Volumens erzeugbar ist und in das Volumen leitbar ist. Das heißt insbesondere, dass das Plasma außerhalb des Volumens erzeugbar ist und das mittels des Plasmas gebildete plasmaaktivierte Medium in das Volumen leitbar ist.

In dieser Ausgestaltung lässt sich somit das Plasma bzw. plasmaaktiviertes Medium entfernt von der zu behandelnden Oberfläche erzeugen und der Oberfläche zuleiten. Hierbei wird also ein Gerät zur Unterdruckwundtherapie mit einer, z.B. über eine Schlauchleitung, mit dem erfindungsgemäßen System zur Behandlung von Oberflächen, insbesondere dem Vakuumverband, verbundenen externen Plasmaquelle kombiniert. Die zweite Einrichtung zur Plasmaerzeugung, also die externe Plasmaquelle, kann dabei dazu eingerichtet sein, eine dielektrisch behinderte Entladung zu erzeugen. Alternativ dazu kann es sich bei der zweiten Einrichtung zur Plasmaerzeugung z.B. auch um eine, insbesondere im kHz-, MHz- oder GHz-Bereich angesteuerte, Jet-Plasmaquelle handeln.

Insbesondere kann das System bei der beschriebenen Ausführungsform dazu eingerichtet sein, mittels einer, vorzugsweise mikroprozessor-basierten, Prozesssteuerung gleichzeitig oder im Wechsel ein durch die Plasmaquelle erzeugtes plasmaaktiviertes Medium (z.B. Gas oder Aerosol) abzusaugen oder zuzuführen.

Die erste Einrichtung zur Plasmaerzeugung, also die interne Plasmaquelle und die zweite Einrichtung zur Plasmaerzeugung, also die externe Plasmaquelle, werden insbesondere alternativ zueinander zur Plasmaerzeugung verwendet. Sie können allerdings auch in Kombination miteinander verwendet werden.

Eine weitere Ausführungsform sieht vor, dass das System zur Behandlung von Oberflächen von Körpern ein poröses Material, insbesondere Schaumstoff, aufweist, welches das Volumen definiert, in dem der Unterdruck erzeugbar ist. Dabei versteht es sich, dass sich das poröse Material bei Anlegen des Unterdrucks zusammenziehen kann, so dass das von dem porösen Material definierte Volumen je nach Vorhandensein eines Unterdrucks schwanken kann.

In einer weiteren Ausführungsform ist vorgesehen, dass das System zur Behandlung von Oberflächen von Körpern eine Kammer aufweist, welche das Volumen definiert, in dem der Unterdruck erzeugbar ist. Dabei ist unter dem Begriff Kammer insbesondere eine Kavität des Systems zu verstehen, welche gemeinsam mit der Oberfläche des entsprechenden zu behandelnden Körpers das besagte Volumen ausbildet.

Das System zur Behandlung von Oberflächen von Körpern kann außerdem eine Unterdruckerzeugungseinrichtung, insbesondere eine Vakuumpumpe, zur Erzeugung eines Unterdrucks in dem Volumen aufweisen, wobei die Unterdruckerzeugungseinrichtung mittels der Anschlusseinrichtung strömungstechnisch mit dem Volumen verbindbar oder verbunden ist.

Gemäß einer weiteren Ausgestaltung ist die Einrichtung zur Plasmaerzeugung, insbesondere die erste oder die zweite Einrichtung zur Plasmaerzeugung, dazu ausgebildet, eine dielektrisch behinderte Entladung zu erzeugen.

In einer weiteren Ausführungsform ist die Einrichtung zur Plasmaerzeugung aus flexiblen Materialien gefertigt, die dazu ausgebildet sind, an der Oberfläche des Körpers anzuliegen, wobei die flexiblen Materialien zumindest einen Teil des Volumens ausbilden.

Ferner sieht eine weitere Ausführungsform vor, dass das System zur Behandlung von Oberflächen von Körpern eine erste Schicht aus flexiblen Materialien aufweist, die dazu ausgebildet ist, an der Oberfläche des Körpers anzuliegen, und dass das System eine zweite Schicht aus flexiblen Materialien aufweist, wobei die Einrichtung zur Plasmaerzeugung flächig ausgebildet ist und zwischen der ersten Schicht und der zweiten Schicht angeordnet ist, und wobei die erste Schicht, die Einrichtung zur Plasmaerzeugung und die zweite Schicht das Volumen ausbilden, in dem der Unterdruck erzeugbar ist.

Die Einrichtung zur Plasmaerzeugung kann wenigstens eine Innenelektrode und wenigstens eine geerdete Außenelektrode aufweisen.

Dabei kann die Außenelektrode ein elektrisch leitendes textiles, insbesondere gewebtes, Material umfassen, bzw. aus diesem bestehen.

Gemäß einer weiteren Ausführungsform kann die Außenelektrode elektrisch leitende Gaze umfassen bzw. aus dieser bestehen.

Eine weitere Ausgestaltung der Erfindung sieht vor, dass die Einrichtung zur Plasmaerzeugung eine Mehrzahl von Abstandselementen aus einem elektrisch isolierenden Material aufweist, wobei die Abstandselemente einen Abstand zwischen der Innenelektrode und der Außenelektrode definieren.

Die beschriebenen Ausführungsformen der Einrichtung zur Plasmaerzeugung eignen sich besonders gut als flache flexible Plasmaquelle und somit zur Verwendung als interne Plasmaquelle in dem erfindungsgemäßen System zur Behandlung von Oberflächen von Körpern.

Weiterhin ist gemäß einer Ausführungsform vorgesehen, dass das System zur Behandlung von Oberflächen von Körpern eine, insbesondere transparente, Folie zur Abdichtung und Abdeckung des Systems gegen die Umgebung aufweist, wobei insbesondere die Folie wenigstens eine Öffnung aufweist, an der die Anschlusseinrichtung angeschlossen ist, so dass die Unterdruckerzeugungseinrichtung durch die Öffnung mit dem Volumen in strömungstechnischer Verbindung steht, in dem der Unterdruck erzeugbar ist.

Ein zweiter Aspekt der Erfindung betrifft ein Verfahren zur Behandlung von Oberflächen von Geweben, nämlich zur Behandlung von Oberflächen menschlichen oder tierischen Gewebes außerhalb des menschlichen bzw. tierischen Körpers, wobei ein Gewebe mit einer zu behandelnden Oberfläche bereitgestellt wird, ein System zur Behandlung von Oberflächen von Körpern nach dem ersten Aspekt der Erfindung bereitgestellt wird, ein Unterdruck in einem an der Oberfläche des Gewebes positionierten Volumen erzeugt wird, insbesondere mittels einer mit der Anschlusseinrichtung verbundenen Unterdruckerzeugungseinrichtung des Systems zur Behandlung von Oberflächen, und das Volumen zumindest teilweise mit Plasma bzw. plasmaaktiviertem Medium gefüllt wird, wobei das Plasma oder das plasmaaktivierte Medium insbesondere mittels der Einrichtung zur Plasmaerzeugung des Systems zur Behandlung von Oberflächen erzeugt wird.

Das bedeutet, dass das Verfahren nicht im oder am menschlichen Körper, sondern außerhalb des menschlichen bzw. tierischen Körpers durchgeführt wird.

Gemäß einer Ausführungsform des Verfahrens wird das Volumen zeitlich alternierend mit der Unterdruckerzeugung mit dem Plasma oder dem plasmaaktivierten Medium gefüllt. Dabei kann das Plasma bzw. das plasmaaktivierte Medium vorzugsweise zeitlich alternierend mit der Unterdruckerzeugung in dem Volumen positioniert werden. Zudem kann mittels der ersten Einrichtung zur Plasmaerzeugung die Funktion eines Sensors ausgeübt werden, insbesondere dann, wenn diese erste Einrichtung zur Plasmaerzeugung nicht zur Erzeugung von Plasma eingesetzt wird.

Bei der Verwendung der Elektroden der Plasmaquelle zu Sensorzwecken im Wundbereich, z.B. zur Kontrolle von Temperatur, Druck, Feuchtigkeit oder pH-Wert kann beispielsweise von einem aktiven Plasmaerzeugungs-Modus in einen Sensor-Modus umgeschaltet werden. Hierfür kann z.B. eine mikroprozessor-basierte Prozesssteuerung verwendet werden.

Bei dem Verfahren kann das Plasma oder das plasmaaktivierte Medium direkt in dem an der Oberfläche des Gewebes positionierten Volumen erzeugt werden und/oder extern erzeugt werden und dem besagten Volumen zugeleitet werden.

Ein dritter Aspekt der Erfindung betrifft die Verwendung eines Systems zur Behandlung von Oberflächen von Körpern nach dem ersten Aspekt der Erfindung zur Behandlung von Oberflächen von Geweben, insbesondere von menschlichen oder tierischen Geweben. Dabei handelt es sich bei dem System insbesondere um einen Vakuumverband.

Ein vierter Aspekt der Erfindung betrifft ein Verfahren zur Behandlung von Oberflächen von menschlichen oder tierischen Gewebes, wobei ein menschliches oder tierisches Gewebe mit einer zu behandelnden Oberfläche bereitgestellt wird, ein System zur Behandlung von Oberflächen von Körpern nach dem ersten Aspekt der Erfindung bereitgestellt wird, ein Unterdruck in einem an der Oberfläche des Gewebes positionierten Volumen erzeugt wird und das Volumen zumindest teilweise mit Plasma bzw. plasmaaktiviertem Medium gefüllt wird. Dabei handelt es sich bei dem System insbesondere um einen Vakuumverband.

Gemäß einer Ausführungsform des Verfahrens wird das Volumen zeitlich alternierend mit der Unterdruckerzeugung mit dem Plasma oder dem plasmaaktivierten Medium gefüllt. Dabei kann das Plasma bzw. das plasmaaktivierte Medium vorzugsweise zeitlich alternierend mit der Unterdruckerzeugung in dem Volumen positioniert werden. Zudem kann mittels der ersten Einrichtung zur Plasmaerzeugung die Funktion eines Sensors ausgeübt werden, insbesondere dann, wenn diese erste Einrichtung zur Plasmaerzeugung nicht zur Erzeugung von Plasma eingesetzt wird.

Bei dem Verfahren kann das Plasma oder das plasmaaktivierte Medium direkt in dem an der Oberfläche des Gewebes positionierten Volumen erzeugt werden und/oder extern erzeugt werden und dem besagten Volumen zugeleitet werden.

Weitere Einzelheiten und Vorteile der vorliegenden Erfindung ergeben sich aus der nachfolgenden Beschreibung von Ausführungsbeispielen. Mit den folgenden, in einer Reihe von Zeichnungen dargestellten, Ausführungsbeispielen werden das Konzept der Erfindung sowie der schematische Aufbau und die Handhabung des Gerätesystems detailliert erläutert. Für die Kennzeichnung der einzelnen Elemente des Aufbaus der Vorrichtungen werden die unten angegebenen Bezugszeichen verwendet.

Dabei zeigen:
- Figur 1: eine Querschnittsdarstellung eines erfindungsgemäßen Systems zur Behandlung von Oberflächen;
- Figur 2: eine schematische Darstellung einer ersten Ausführungsform des erfindungsgemäßen Systems mit interner Plasmaquelle;
- Figur 3: eine Explosionsdarstellung der ersten Ausführungsform des erfindungsgemäßen Systems mit interner Plasmaquelle;
- Figur 4: eine perspektivische Ansicht einer Ausgestaltungsform einer internen Plasmaquelle gemäß der Erfindung;
- Figur 5: eine Explosionsdarstellung der in Figur 4 gezeigten Ausgestaltungsform der internen Plasmaquelle;
- Figur 6: eine schematische Darstellung einer zweiten Ausführungsform des erfindungsgemäßen Systems mit externer Plasmaquelle;
- Figur 7: eine Querschnittsdarstellung einer externen DBE-basierten Plasmaquelle.

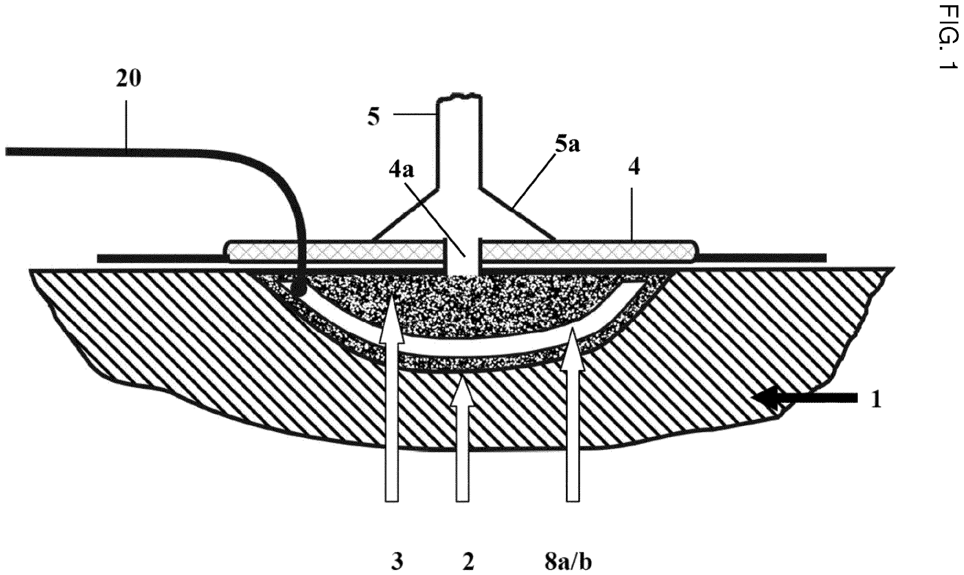

Die Figur 1 zeigt die übliche prinzipielle Anordnung der wesentlichen Komponenten eines als Vakuumverband in einer Wunde eines biologischen Gewebes 1 ausgestalteten erfindungsgemäßen Systems mit einer, zusätzlich zwischen zwei Schaumstoffwundauflagen 2,3 eingebetteten, flexiblen, flächigen DBE-basierten Plasmaquelle 8a, die zusätzlich als Sensorsystem 8b für Wund-Monitoring verwendet werden kann. Die Wunde ist von einer, z.B. transparenten Folie 4 abgedeckt und gegen die Umgebung abgedichtet. Dabei liegt die Folie 4 dichtend an der Wundauflage 3 und der die Wunde umgebenden Körperoberfläche an.

Die Folie 4 weist eine Öffnung 4a und eine über der Öffnung 4a angeordnete Anschlusseinrichtung 5a zum strömungstechnischen Anschluss einer Unterdruckerzeugungseinrichtung zur Erzeugung eines Unterdrucks in dem von den Schaumstoffwundauflagen 2,3 gebildeten Volumen, z.B. über einen Drainageschlauch 5, auf. Der Drainageschlauch 5 kann gleichzeitig zum Absaugen von Wundflüssigkeit verwendet werden.

Die Plasmaquelle 8a bzw. das Sensorsystem 8b ist mit einer elektrischen Zuleitung 20 zum Anschluss an eine Spannungsversorgung 10 und/oder ein Mess- und Steuergerät 9 für das Wund-Monitoring verbunden. Mittels der dargestellten Plasmaquelle 8a kann unmittelbar in dem Volumen, in dem der Unterdruck erzeugt wird, also in diesem Fall in dem von den Schaumstoffwundauflagen 2,3 gebildeten Volumen, ein Plasma generiert werden.

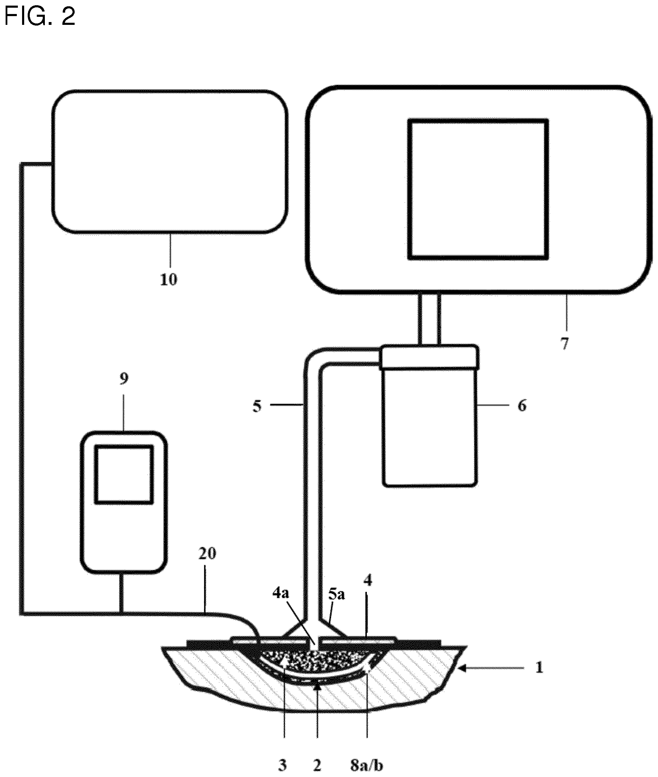

Ein Schema des Gesamtaufbaus des kombinierten Geräte-Systems gemäß einer ersten Ausführungsform mit im Vakuumverband eingebetteter interner DBE-basierter Plasmaquelle 8a ist in der Figur 2 dargestellt. Der Vakuumverband ist dabei zum Zweck der Evakuierung und/oder zur Absaugung von Wundsekret in der üblichen Weise über eine Anschlusseinrichtung 5a und über einen Drainageschlauch 5 und einen Auffangbehälter 6 für Wundsekret an ein Unterdruck-Steuergerät 7 mit Vakuumpumpe angeschlossen, während die Kombination aus einer DBE-basierten Plasmaquelle 8a und einem Sensorsystem 8b über entsprechende elektrische Zuleitungen 20 einerseits mit einem Mess- und Steuergerät 9 für das Wund-Monitoring mittels des Sensorsystems 8b und andererseits mit der Spannungsversorgung 10 für die Plasmaquelle 8a verbunden ist.

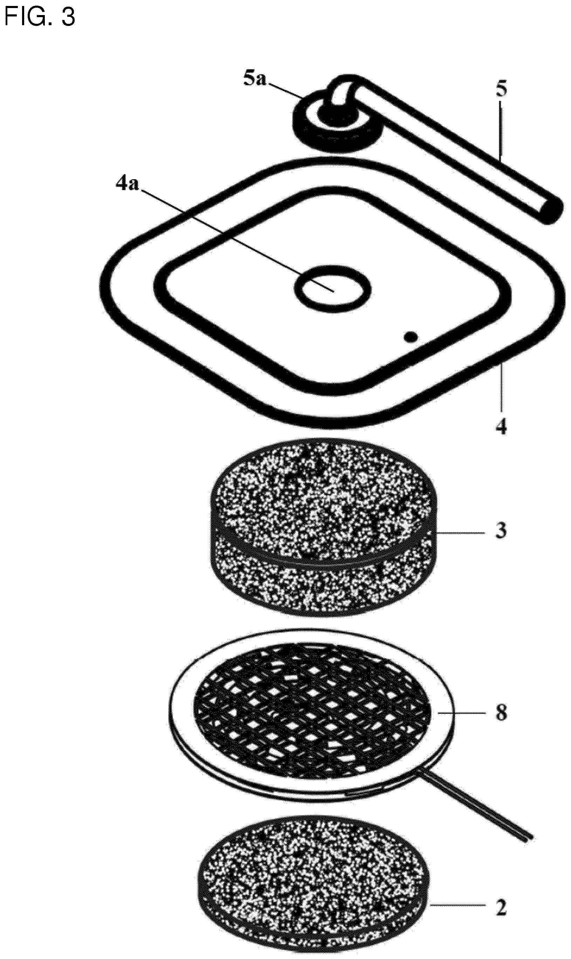

Die in der Figur 3 gezeigte Explosionszeichnung der als Vakuumverband ausgestalteten ersten Ausführungsform des erfindungsgemäßen Systems zur Behandlung von Oberflächen mit interner Plasmaquelle 8 soll noch einmal anschaulich die Sandwichanordnung der dünnen Schaumstoff-Wundauflage 2, die direkt auf der Wunde liegt, der Kombination 8 aus einer DBE-basierten Plasmaquelle und einem Sensorsystem, des darüber liegenden Teils der Schaumstoff-Wundauflage 3 und der transparenten Folie 4 zur Abdichtung und Abdeckung mit der Anschlusseinrichtung 5a für den Drainageschlauch 5 in dem Vakuumverband demonstrieren.

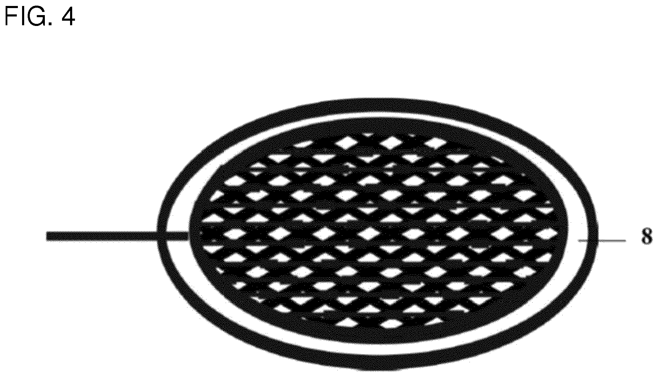

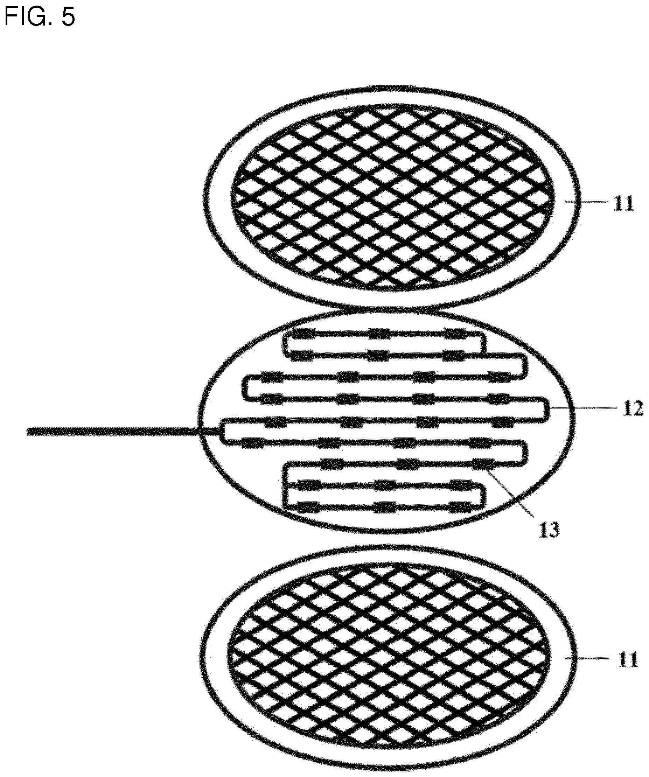

Die Figuren 4 und 5 zeigen den prinzipiellen Aufbau einer Ausgestaltung der aus verschiedenen Lagen zusammengesetzten Kombination 8 aus einer DBE-basierten Plasmaquelle und einem Sensorsystem im Zusammenbau (Figur 4) und als Explosionszeichnung (Figur 5).

Ein zentral mäanderförmig angeordneter, isolierter elektrischer Leiter 12 dient dabei wahlweise als Teil eines Sensorsystems 8b oder als, mit einem Dielektrikum bedeckte, Hochspannungselektrode, an die sich in einem definierten, durch die Abstandselemente 13 aus Isoliermaterial definierten, Abstand, von beiden Seiten jeweils eine, aus elektrisch leitender Gaze oder elektrisch leitendem textilem, insbesondere gewebten, Material bestehende, geerdete Außenelektrode 11 anschmiegt, die ebenfalls wahlweise als Teil des Sensorsystems 8b zum Wund-Monitoring genutzt werden kann. Anstelle der mäanderförmigen Anordnung des isolationsmaterialummantelten elektrischen Leiters 12 ist auch eine spiralförmige Anordnung oder irgendeine Anordnung möglich, mit der möglichst flächendeckend in einer Ebene durch entsprechenden Elektrodenverlauf Plasma erzeugt werden kann.

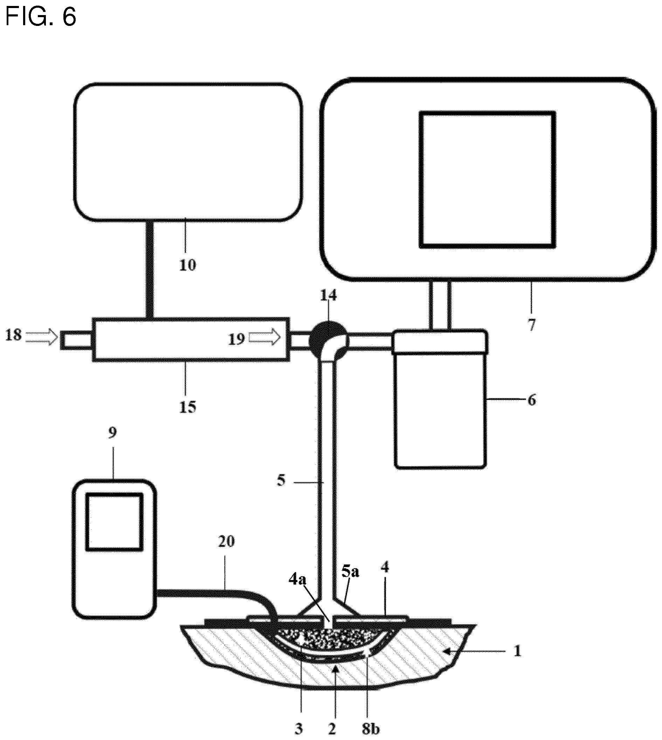

In der Figur 6 ist eine zweite Ausführungsform des erfindungsgemäßen Systems zur Behandlung von Oberflächen, also eine weitere Möglichkeit der Kombination eines Unterdrucktherapie-Gerätes mit einer externen Plasmaquelle 15 dargestellt. Im Unterschied zu der in der Figur 2 dargestellten ersten Ausführungsform wird hier anstelle der internen DBE-basierten Plasmaquelle 8a eine externe DBE-basierte Plasmaquelle 15 verwendet. Dabei wird in dem hier dargestellten Ausführungsbeispiel das durch die Plasmaquelle 15 aus dem zugeführten Gas 18 erzeugte plasmabehandelte Gas 19 über einen Dreiwegehahn 14 zeitweise in definierter Menge über den Drainageschlauch 5 und die Anschlusseinrichtung 5a dem Vakuumverband und damit dem Volumen, in dem der Unterdruck erzeugt wird, zugeführt.

Nach einer definierten Einwirkungszeit wird dann wieder über den Dreiwegehahn 14 die Absaugung mit dem Unterdrucktherapie-Gerät bzw. der Unterdruckerzeugungseinrichtung, z.B. einer Vakuumpumpe als Teil des Unterdrucksteuergeräts 7, realisiert. In diesem Fall ist die in der Figur 4 dargestellte Elektrodenanordnung ebenfalls in dem Vakuumverband integriert, dient aber hier nicht als Plasmaquelle sondern nur als Sensorsystem 8b in Verbindung mit dem Mess- und Steuergerät 9 für Wund-Monitoring.

Als externe Plasmaquelle 15 können hierbei vielfältige Ausgestaltungen verwendet werden.

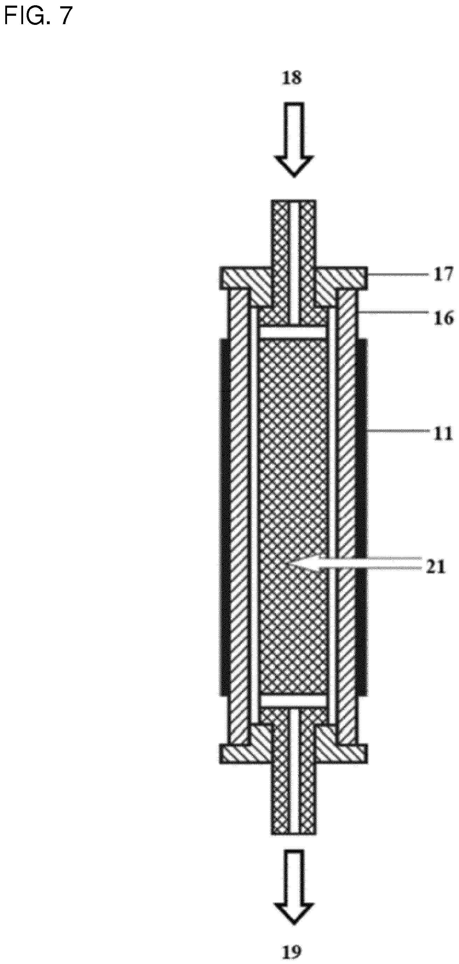

In der Figur 7 ist beispielhaft der Aufbau einer koaxialen Anordnung einer DBD-basierten Plasmaquelle schematisch als Schnittzeichnung dargestellt. In diesem Fall ist die Hochspannung führende Innenelektrode 21 ein Metallzylinder, der von einem, als Dielektrikum dienenden mit einer geerdeten Außenelektrode 11 beschichteten, Glasrohr 16 umgeben ist. Der enge Spalt zwischen dem Glasrohr 16 und der Innenelektrode 12, der durch die Zentrier- und Dichtringe 17 nach außen abgedichtet ist, dient als Gasraum, in dem sich das Plasma ausbildet.

### Bezugszeichenliste

| | |
|---|---|
| 1 | biologisches Gewebe |
| 2 | Schaumstoff-Wundauflage, Teil A |
| 3 | Schaumstoff-Wundauflage, Teil B |
| 4 | Folie, insbesondere transparente Folie zur Abdichtung und Abdeckung |
| 4a | Öffnung |
| 5 | Drainageschlauch |
| 5a | Anschlusseinrichtung |
| 6 | Auffangbehälter für Wundsekret |
| 7 | Unterdruck-Steuergerät mit Vakuumpumpe |
| 8 | Erste Einrichtung zur Plasmaerzeugung, insbesondere flexible, flächige DBE-basierte Plasmaquelle bzw. Kombination aus Plasmaquelle und Sensorsystem |
| 8a | Erste Einrichtung zur Plasmaerzeugung, insbesondere flexible, flächige DBE-basierte Plasmaquelle, |
| 8b | Sensorsystem für Wund-Monitoring |
| 9 | Mess- und Steuergerät für Wund-Monitoring |
| 10 | Spannungsversorgung für die Plasmaquelle |
| 11 | geerdete Außenelektrode |
| 12 | Hochspannung führende, isolationsmaterialummantelte Innenelektrode |
| 13 | Abstandselemente aus Isoliermaterial |
| 14 | Dreiwegehahn |
| 15 | Zweite Einrichtung zur Plasmaerzeugung, insbesondere externe DBE-basierte Plasmaquelle |
| 16 | Glasrohr |
| 17 | Zentrier- und Dichtring |
| 18 | Gas (z.B. Luft) |
| 19 | plasmabehandeltes Gas |
| 20 | elektrische Zuleitungen |
| 21 | metallische Innenelektrode |

## Patentansprüche

1. System zur Behandlung von Oberflächen von Körpern, insbesondere Vakuumverband, umfassend
- wenigstens eine Anschlusseinrichtung (5a) zum strömungstechnischen Anschluss einer Unterdruckerzeugungseinrichtung zur Erzeugung eines Unterdrucks in einem an der Oberfläche eines Körpers positionierbaren Volumen sowie
- wenigstens eine Einrichtung zur Plasmaerzeugung (8,8a,15), mit der das Volumen, in welchem der Unterdruck erzeugbar ist, zumindest teilweise mit Plasma oder einem plasmaaktivierten Medium gefüllt werden kann.
wobei das System eine erste Einrichtung zur Plasmaerzeugung (8,8a) aufweist, die in dem Volumen, in welchem der Unterdruck erzeugbar ist, angeordnet ist, so dass das Plasma und/oder plasmaaktivierte Medium in dem Volumen erzeugbar ist,
**dadurch gekennzeichnet, dass**
die erste Einrichtung zur Plasmaerzeugung (8,8a) als ein Sensorsystem (8b) zur Erfassung wenigstens eines physikalischen Parameters an der Oberfläche des Körpers eingerichtet ist, wobei die Plasmaquelle (8a) der ersten Einrichtung zur Plasmaerzeugung (8) zusätzlich als Sensorsystem (8b) für Wund-Monitoring verwendet werden kann.

2. System zur Behandlung von Oberflächen von Körpern nach Anspruch 1, **dadurch gekennzeichnet, dass** das System eine zweite Einrichtung zur Plasmaerzeugung (15) aufweist, die in Bezug zu dem Volumen, in welchem der Unterdruck erzeugbar ist, extern angeordnet ist und mit einer strömungstechnischen Verbindung mit dem Volumen verbunden ist, so dass das plasmaaktivierte Medium außerhalb des Volumens erzeugbar ist und in das Volumen leitbar ist.

3. System zur Behandlung von Oberflächen von Körpern nach mindestens einem der vorhergehenden Ansprüche, **dadurch gekennzeichnet, dass** das System ein poröses Material, insbesondere Schaumstoff, aufweist, welches das Volumen definiert, in dem der Unterdruck erzeugbar ist.

4. System zur Behandlung von Oberflächen von Körpern nach mindestens einem der vorhergehenden Ansprüche, **dadurch gekennzeichnet, dass** das System eine Kammer aufweist, welche das Volumen definiert, in dem der Unterdruck erzeugbar ist.

5. System zur Behandlung von Oberflächen von Körpern nach mindestens einem der vorhergehenden Ansprüche, **dadurch gekennzeichnet, dass** das System eine Unterdruckerzeugungseinrichtung, insbesondere eine Vakuumpumpe, zur Erzeugung eines Unterdrucks in dem Volumen aufweist, wobei die Unterdruckerzeugungseinrichtung mittels der Anschlusseinrichtung (5a) strömungstechnisch mit dem Volumen verbindbar oder verbunden ist.

6. System zur Behandlung von Oberflächen von Körpern nach mindestens einem der vorhergehenden Ansprüche, **dadurch gekennzeichnet, dass** die Einrichtung zur Plasmaerzeugung (8,8a, 15) dazu ausgebildet ist, eine dielektrisch behinderte Entladung zu erzeugen.

7. System zur Behandlung von Oberflächen von Körpern nach mindestens einem der vorhergehenden Ansprüche, **dadurch gekennzeichnet, dass** die Einrichtung zur Plasmaerzeugung (8,8a,15) wenigstens eine Innenelektrode (12,21) und wenigstens eine geerdete Außenelektrode (11) aufweist.

8. System zur Behandlung von Oberflächen von Körpern nach Anspruch 7, **dadurch gekennzeichnet, dass** die Außenelektrode (11) ein elektrisch leitendes textiles, insbesondere gewebtes, Material umfasst, bzw. aus diesem besteht.

9. System zur Behandlung von Oberflächen von Körpern nach Anspruch 7 oder 8, wobei die Außenelektrode (11) elektrisch leitende Gaze umfasst bzw. aus dieser besteht.

10. System zur Behandlung von Oberflächen von Körpern nach mindestens einem der Ansprüche 7 bis 9, **dadurch gekennzeichnet, dass** die Einrichtung zur Plasmaerzeugung (8,8a,15) eine Mehrzahl von Abstandselementen (13) aus einem elektrisch isolierenden Material aufweist, wobei die Abstandselemente (13) einen Abstand zwischen der Innenelektrode (12,21) und der Außenelektrode (11) definieren.

11. System zur Behandlung von Oberflächen von Körpern nach mindestens einem der vorhergehenden Ansprüche, **dadurch gekennzeichnet, dass** das System eine, insbesondere transparente, Folie (4) zur Abdichtung und Abdeckung des Systems gegen die Umgebung aufweist, wobei insbesondere die Folie (4) wenigstens eine Öffnung (4a) aufweist, an der die Anschlusseinrichtung (5a) angeschlossen ist, so dass die Unterdruckerzeugungseinrichtung durch die Öffnung (4a) mit dem Volumen in strömungstechnischer Verbindung steht, in dem der Unterdruck erzeugbar ist.

12. Verfahren zur Behandlung von Oberflächen von Gewebe, nämlich zur Behandlung von Oberflächen menschlichen oder tierischen Gewebes außerhalb des menschlichen bzw. tierischen Körpers, wobei
i) ein Gewebe mit einer zu behandelnden Oberfläche bereitgestellt wird,
ii) ein System zur Behandlung von Oberflächen von Körpern nach mindestens einem der Ansprüche 1 bis 11 bereitgestellt wird,
iii) ein Unterdruck in einem an der Oberfläche des Gewebes positionierten Volumen erzeugt wird,
v) das Volumen zumindest teilweise mit Plasma bzw. plasmaaktiviertem Medium gefüllt wird.

13. Verfahren zur Behandlung von Oberflächen von Gewebe nach Anspruch 12, wobei das Volumen zeitlich alternierend mit der Unterdruckerzeugung mit dem Plasma oder dem plasmaaktivierten Medium gefüllt wird.

## Claims

1. A system for treating surfaces of bodies, in particular vacuum dressing, comprising
- at least one connection device (5a) for the fluidic connection of a negative pressure generating device for generating a negative pressure in a volume which can be positioned on the surface of a body, and
- at least one device for generating plasma (8, 8a, 15), with which the volume in which the negative pressure can be generated can be at least partially filled with plasma or a plasma-activated medium.
wherein the system comprises a first device for generating plasma (8, 8a) which is arranged in the volume in which the negative pressure can be generated, so that the plasma and/or plasma-activated medium can be generated in the volume,
**characterized in that**
the first device for plasma generation (8, 8a) is set up as a sensor system (8b) for detecting at least one physical parameter on the surface of the body, wherein the plasma source (8a) of the first device for plasma generation (8) can additionally be used as a sensor system (8b) for wound monitoring.

2. The system for treating surfaces of bodies according to claim 1, **characterized in that** the system comprises a second device for plasma generation (15) which is arranged externally in relation to the volume in which the negative pressure can be generated and is connected to the volume by a fluidic connection, so that the plasma-activated medium can be generated outside the volume and can be conducted into the volume.

3. The system for treating surfaces of bodies according to at least one of the preceding claims, **characterized in that** the system comprises a porous material, in particular foam, which defines the volume in which the negative pressure can be generated.

4. The system for treating surfaces of bodies according to at least one of the preceding claims, **characterized in that** the system comprises a chamber which defines the volume in which the negative pressure can be generated.

5. The system for treating surfaces of bodies according to at least one of the preceding claims, **characterized in that** the system comprises a negative pressure generating device, in particular a vacuum pump, for generating a negative pressure in the volume, wherein the negative pressure generating device is fluidically connectable or connected to the volume by means of the connecting device (5a).

6. The system for treating surfaces of bodies according to at least one of the preceding claims, **characterized in that** the device for plasma generation (8, 8a, 15) is designed to generate a dielectrically impeded discharge.

7. The system for treating surfaces of bodies according to at least one of the preceding claims, **characterized in that** the device for plasma generation (8, 8a, 15) comprises at least one inner electrode (12, 21) and at least one earthed outer electrode (11).

8. The system for treating surfaces of bodies according to claim 7, **characterized in that** the outer electrode (11) comprises or consists of an electrically conductive textile, in particular woven, material.

9. The system for treating surfaces of bodies according to claim 7 or 8, wherein the outer electrode (11) comprises or consists of electrically conductive gauze.

10. The system for treating surfaces of bodies according to at least one of claims 7 to 9, **characterized in that** the device for plasma generation (8, 8a, 15) comprises a plurality of spacer elements (13) made of an electrically insulating material, wherein the spacer elements (13) define a distance between the inner electrode (12, 21) and the outer electrode (11).

11. The system for treating surfaces of bodies according to at least one of the preceding claims, **characterized in that** the system comprises a film (4), in particular a transparent film, for sealing and covering the system from the environment, wherein in particular the film (4) comprises at least one opening (4a) to which the connecting device (5a) is connected, so that the negative pressure generating device is in fluidic connection through the opening (4a) with the volume in which the negative pressure can be generated.

12. A method for treating tissue surfaces, namely for treating surfaces of human or animal tissue outside the human or animal body, wherein
i) a tissue with a surface to be treated is provided,
ii) a system for treating surfaces of bodies according to at least one of claims 1 to 11 is provided,
iii) a negative pressure is generated in a volume positioned on the surface of the tissue,
v) the volume is at least partially filled with plasma or plasma-activated medium.

13. The method for treating tissue surfaces according to claim 12, wherein
the volume is filled with the plasma or the plasma-activated medium alternately with the generation of negative pressure.

## Revendications

1. Système de traitement de surfaces de corps, notamment pansement par aspiration, comprenant
- au moins un dispositif de raccord (5a) pour le raccord technique d'écoulement d'un dispositif de génération de pression négative en vue de la génération d'une pression négative dans un volume pouvant être positionné à la surface d'un corps ainsi que
- au moins un dispositif pour la génération de plasma (8, 8a, 15) avec lequel le volume dans lequel la pression négative peut être générée peut être au moins partiellement rempli de plasma ou d'un milieu activé par plasma,
dans lequel le système présente un premier dispositif pour la génération de plasma (8, 8a) qui est disposé dans le volume dans lequel la pression négative peut être générée de sorte que le plasma et/ou milieu activé par plasma peut être généré dans le volume,
**caractérisé en ce que**
le premier dispositif pour la génération de plasma (8, 8a) est configuré en tant que système de capteur (8b) pour la détection d'au moins un paramètre physique à la surface du corps, dans lequel la source de plasma (8a) du premier dispositif pour la génération de plasma (8) peut en outre être utilisée en tant que système de capteur (8b) pour la surveillance de plaie.

2. Système de traitement de surfaces de corps selon la revendication 1, **caractérisé en ce que** le système présente un second dispositif pour la génération de plasma (15) qui est disposé de manière externe par rapport au volume dans lequel la pression négative peut être générée et est connecté avec une connexion technique d'écoulement au volume de sorte que le milieu activé par plasma peut être généré en dehors du volume et peut être conduit dans le volume.

3. Système de traitement de surfaces de corps selon au moins une des revendications précédentes, **caractérisé en ce que** le système présente un matériau poreux, notamment de la mousse, qui définit le volume dans lequel la pression négative peut être générée.

4. Système de traitement de surfaces de corps selon au moins une des revendications précédentes, **caractérisé en ce que** le système présente une chambre qui définit le volume dans lequel la pression négative peut être générée.

5. Système de traitement de surfaces de corps selon au moins une des revendications précédentes, **caractérisé en ce que** le système présente un dispositif de génération de pression négative, notamment une pompe à vide, pour la génération d'une pression négative dans le volume, dans lequel le dispositif de génération de pression négative peut être connecté ou est connecté en matière de technique d'écoulement au volume au moyen du dispositif de raccord (5a).

6. Système de traitement de surfaces de corps selon au moins une des revendications précédentes, **caractérisé en ce que** le dispositif pour la génération de plasma (8, 8a, 15) est réalisé pour générer une décharge à barrière diélectrique.

7. Système de traitement de surfaces de corps selon au moins une des revendications précédentes, **caractérisé en ce que** le dispositif pour la génération de plasma (8, 8a, 15) présente au moins une électrode interne (12, 21) et au moins une électrode externe mise à la terre (11).

8. Système de traitement de surfaces de corps selon la revendication 7, **caractérisé en ce que** l'électrode externe (11) comprend un matériau textile électriquement conducteur, notamment tissé, ou se compose de celui-ci.

9. Système de traitement de surfaces de corps selon la revendication 7 ou 8, **caractérisé en ce que** l'électrode externe (11) comprend de la gaze électriquement conductrice ou se compose de celle-ci.

10. Système de traitement de surfaces de corps selon au moins une des revendications 7 à 9, **caractérisé en ce que** le dispositif pour la génération de plasma (8, 8a, 15) présente une pluralité d'éléments d'écartement (13) en un matériau électriquement isolant, dans lequel les éléments d'écartement (13) définissent un écart entre l'électrode interne (12, 21) et l'électrode externe (11).

11. Système de traitement de surfaces de corps selon au moins une des revendications précédentes, **caractérisé en ce que** le système présente une feuille (4), notamment transparente, pour l'étanchéification et le recouvrement du système vis-à-vis de l'environnement, dans lequel la feuille (4) présente notamment au moins une ouverture (4a) à laquelle le dispositif de raccord (5a) est raccordé de sorte que le dispositif de génération de pression négative est en connexion technique d'écoulement au volume, dans lequel la pression négative peut être générée, à travers l'ouverture (4a).

12. Procédé de traitement de surfaces de corps, à savoir de traitement de surfaces de tissu humain ou animal en dehors du corps humain ou animal, dans lequel
i) un tissu avec une surface à traiter est mis à disposition,
ii) un système de traitement de surfaces de corps selon au moins une des revendications 1 à 11 est mis à disposition,
iii) une pression négative est générée dans un volume positionné à la surface du tissu,
v) le volume est au moins partiellement rempli de plasma ou milieu activé par plasma.

13. Procédé de traitement de surfaces de tissu selon la revendication 12, dans lequel le volume est rempli du plasma ou du milieu activé par plasma en alternance dans le temps avec la génération de pression négative.
